# EUROPEAN PATENT APPLICATION

(11) **EP 3 501 503 A1**
(43) Date of publication of application: **26.06.2019**
(21) Application number: 17210110.7
(22) Date of filing: 22.12.2017
(51) Int. Cl.: A61K 9/20, A61K 39/00, A61K 9/28

(54) **SOLID DELIVERY COMPOSITION**

(71) Applicant: Cosmo Technologies Ltd., Dublin 2 (IE)
(72) Inventor: Macelloni, Cristina, D2 Dublin (IT); Longo, Luigi Maria, D2 Dublin (IT); Rossi, Silvia, 27100 Pavia (IT); Franca, Ferrari, 27100 Pavia (IT)
(74) Representative: FRKelly

(57) **Abstract**

The present invention provides an innovative combination of biomaterials, multifunctional polymers, able to structure themselves at the site of action. The innovative combination of biomaterials of the invention is formulated in a solid composition and can be used as a versatile delivery vehicle or carrier in many pharmaceutical fields. The solid composition comprising the combination of biomaterials of the invention is provided to target delivery for medical purposes in humans.

## Description

The present invention provides an innovative combination of biomaterials, multifunctional polymers, able to structure themselves at the site of action for the achievement of a potential bio-adhesion in vivo and a better control of the product release. The innovative combination of biomaterials of the invention is formulated in a solid composition and can be used as a versatile delivery vehicle or carrier in many pharmaceutical fields. The solid composition comprising the combination of biomaterials of the invention is provided to target delivery for medical purposes in humans.

The compositions of the invention can contain active ingredients such as those belonging to the therapeutic classes of proteins, peptides, monoclonal antibodies, anti- TNFs, immune modulators, cytokines, chemo-cytokines, anti-neoplastics, analgesics, anti-inflammatories, antibiotics, antiadrenergic drugs, local anesthetics, non steroid anti-inflammatory drugs, steroid anti-inflammatory drugs, anti-diarrheals drugs, intestinal anti-inflammatories, peripheral opioid receptor antagonists, laxatives, spasmolytics, 3-hydroxy-3-methylglutaryl-coenzyme A (HMG-CoA) reductase inhibitors, bile acid sequestrants and are suitable for oral target administration in view of systemic activity or for acting topically at some areas of the human body, preferably of the gastrointestinal tract.

### BACKGROUND

Targeted drug delivery enables an active ingredient to be selectively targeted or delivered only to its site of action or absorption and not to the non-target organs or tissues or cells. It is a method of delivering medication to a patient in a manner that increases the concentration of the medication in some parts of the body relative to others. This modified disposition of the active ingredient can improve the drug efficacy and reduces its side effects due to product accumulation in some organ or unbalanced PK profile.

Improvements in drug delivery systems can assist problems such as biological instability, dosage form solubility, biopharmaceutical low absorption, low specificity, short half life and low volume of distribution.

A delivery system must be nontoxic, biocompatible, biodegradable and stable. It should have a controllable and predicate rate of release of active ingredient. A minimal leakage during transit is sometime recommended.

Effective target delivery simplifies administration protocols and reduces toxicity. It allows drugs to be administered in smaller doses and can enhance absorption of small molecules and biomolecules such as peptides and particulates if effectively delivered at the target organ.
Targeting delivery can be achieved with a carrier system or vehicle. The carrier or vehicle is designed to safely transport the loaded active ingredient to pre-selected sites.
Suggested carrier systems have included polymers, microcapsules, micro-particles, lipoproteins, liposomes and micelles.

In recent years there has been a significant increase in available strategies for site-specific delivery, especially in the gastrointestinal tract both to maximize a therapeutic response and to reduce side effects of very effective drugs.

Targeted delivery requires a multidisciplinary approach to research, involving contributions from polymer and pharmaceutical scientists and from experts in gastroenterology and product evaluation. Site-specific delivery is designed to deliver medication to a patient in a manner that increases the concentration of the medication in some parts of the body relative to others. This means of delivery is largely founded on bio-material mediated drug delivery in order to combat the downfalls of conventional drug delivery. Bio-material based delivery vehicles would be loaded with drugs and targeted to specific parts of the body thereby avoiding interaction with healthy tissue. The goal of a targeted drug delivery system is to prolong, localize, target and have a protected drug interaction with a target tissue or organ in the body. The advantages to the targeted release system are the possibility of obtaining a topical effect limited to a specific body organ or district, reduction in the frequency of the dosages taken by the patient, having a more uniform effect of the drug, reduction of drug side-effects, and reduced fluctuation in circulating drug levels.

Targeted drug delivery systems have been developed for GI tract delivery. The drug delivery system must be highly integrated and requires contributes of various disciplines, such as chemists, biologists, and engineers, to join forces to optimize.

EP 0 453 001 discloses a multiparticulate with "reservoir" structure inserted in a hydrophilic matrix. The basic multiparticulate utilizes two coating membranes to decrease the release rate of the active ingredient, a pH dependent membrane with the purpose of gastric protection and a pH-independent methacrylic membrane with the purpose of slowing down the penetration of the aqueous fluid.

WO 95/16451 discloses a composition only formed by a hydrophilic matrix coated with a gastro-resistant film for controlling the dissolution rate of mesalazine.

Small molecules active ingredients and Biological active ingredients, such as protein, antibodies or peptides are easily degraded by the acidic conditions and enzymes of the GI tract and such substances generally do not produce the desired effects since a chain of degradative reactions is immediately scattered which leads to the break-down of the substance in a very short time period. Hence, invasive administration forms, such as injectables, that by-pass the GI districts, have been the elected administration path to deliver a defined amount of a peptide or protein substance to a target site.

WO2008031770A2 describes pharmaceutical compositions for oral or rectal administration of peptide and protein substances in controlled and /or site specific release formulations. The compositions therein aimed to protecting the protein or peptide substance during its gastro-intestinal transit.

Whereas many delivery systems have been proposed, there remains a need for improved systems, especially for controlled delivery of the active ingredient within the targeted site.

The present invention aims to provide an improved drug delivery that not only delivers active ingredient to a target site but also controls and prolongs release of the active ingredient at the target site in order to maximize its efficacy.

Furthermore, there is a need for a delivery system that can be adapted for delivery of different active ingredients and different target sites throughout the GI tract after oral administration.

Additionally, there is the need for a drug delivery system, for oral administration of peptides, proteins or antibodies, which is able to target the release into the GI tract and to protect them from the environment and enzymatic hydrolysis.

### DEFINITIONS

References in the specification to "one embodiment", "an embodiment", "one aspect", "an aspect" and similar indicate that the described embodiment or aspect may include a particular aspect, feature, structure or characteristic. Moreover, such phrases may, but do not necessarily, refer to the same embodiment or aspect referred to in other portions of the specification. Further, when a particular aspect, feature, structure or characteristic is described in connection with an embodiment or aspect, it is within knowledge of a person skilled in the art to affect or connect said aspect, feature, structure or characteristic with other embodiment or aspect, whether or not explicitly described.

The singular forms "a", "an" and "the" include plural references unless the context clearly dictates otherwise. Thus, for example, a reference to "a compound" includes a plurality of such compounds. It is further noted that the claims may be drafted to exclude any optional element. As such, this statement is intended to serve as antecedent basis for the use of exclusive terminology, such as "soley", "only", and the like, in connection with the recitation of claims elements or use of a "negative" limitation.

The term "and/or" means anyone of the items, any combination of the items, or all the items with which this term is associated.

The terms "comprising", "having", "including" and "containing" are to be construed as open-ended terms (i.e. meaning "including, but not limited to") and are to be considered as providing support also for terms as "consist essentially of", "consisting essentially of", "consist of" or "consisting of".

The terms "consist essentially of", "consisting essentially of" are to be construed as a semi-closed terms, meaning that no other ingredients which materially affects the basic and novel characteristics (and optionally physiologically acceptable excipients and/or adjuvants) of the invention are included. The terms "consists of", "consisting of" are to be construed as a closed term.
PEG: Polyethylene glycol.
TRIS: tris(hydroxymethyl)aminomethane.
EDTA: Ethylenediaminetetraacetic acid.
DTPA: diethylenetriamine penta-acetic acid.

Unless indicated otherwise herein, the term "about" is intended to include values, e.g. weight percentages, proximate to the recited range that are equivalent in terms of the functionality of the individual ingredient, the composition, or the embodiment.

A person skilled in the art will recognize that, for any and all purposes, particularly in terms of providing a written description, all ranges recited herein also encompass any and all possible sub-ranges and combinations of sub-ranges thereof, as well as the individual values making up the range, particularly integer values. A recited range includes each specific value, integer, decimal, or identity within the range.

A person skilled in the art will recognize that where members are grouped together in a common manner, such as in a Markush group, the invention encompasses not only the entire group listed as a whole, but each member of the group individually and all possible subgroups of the main group. Additionally, for all purposes, the invention encompasses not only the main group, but also the main group absent one or more of the group members. The invention therefore envisages the explicit exclusion of anyone or more of members of a recited group. Accordingly, provisos may apply to any of the disclosed categories or embodiments whereby anyone or more of the recited elements, species, or embodiments, may be excluded from such categories or embodiments, for example, as used in an explicit negative limitation.

The term "alleviating" refers to relieving the symptoms and/or manifestations of inflammatory and/or degenerative diseases of the gastrointestinal tract.

The term "reducing" refers to decreasing the extent of the damage caused by inflammatory and/or degenerative diseases of the gastrointestinal tract or to decreasing clinical signs or symptoms associated with such damage.

"Body temperature" refers to the level of heat produced and sustained by the body processes. Heat is generated within the body through metabolism of nutrients and lost from the body surface through radiation, convection, and evaporation of perspiration. Heat production and loss are regulated and controlled in the hypothalamus and brainstem. Normal adult body temperature, as measured orally, is 37° C, even though little variations are normally recorded throughout the day.

The term "amphiphilic," as used herein refers to an affinity for lipids and apolar compounds, and an affinity for water.

The term "hydrophilic," as used herein, refers to an affinity for water and poor affinity for lipids and apolar compounds.

The term "lipophilic," as used herein, refers to an affinity for lipids and apolar compounds, and a poor affinity for aqueous fluids.

The term "matrix" as used herein refers to a macroscopically homogeneous structure when ideally cut in all its axes or in all its volume.

The term "controlled release," or "modified release" as used herein in relation to a dosage, describes a dosage form whose drug-release characteristics of time course and/or location are chosen to accomplish therapeutic or convenience objectives not offered by conventional dosage forms such as a solution or an immediate release dosage form. Controlled release (or modified release) solid oral dosage forms include both delayed release drug products, extended release drug products and delayed and extended drug products.

The term "physiologically acceptable excipient" as used herein refers to a substance devoid of any pharmacological effect of its own and which does not produce adverse reactions when administered to a mammal, preferably a human. Physiologically acceptable excipients are well known in the art and are disclosed, for instance in the Handbook of Pharmaceutical Excipients, sixth edition 2009, herein incorporated by reference.

The term "gastro-resistant coating," as used herein, refers to a coating that allows a composition, such as a solid oral dosage form such as a tablet, to pass through the gastric tract, such as the stomach, without being damaged or eroded and which then beings dissolving or eroding, or dissolves or erodes substantially or completely, with the consequent release of the active substance, or a pharmaceutically acceptable salt thereof, comprising the dosage form, such as when the environmental conditions change as, for example, when the pH of the gastrointestinal tract changes.

### DISCLOSURE OF THE INVENTION

According to the present invention there is provided a solid composition for targeted and controlled release of an active substance, the composition comprising:
at least one active substance to be delivered;
at least one thermo-responsive polymer;
at least one ion-sensitive polymer; and
at least one lipophilic compound.

According to one embodiment the composition comprises:
the at least one active substance to be delivered; and
a hydrophilic matrix comprising a combination of the at least one thermo-responsive polymer and the at least one ion-sensitive polymer dispersed in the at least one lipophilic compound.

The composition can further comprise a resin in which the hydrophilic matrix associated with at least one lipophilic compound is dispersed.

Other optional substituents may include bio-adhesive polymers, an amphiphilic compound and other physiologically acceptable excipients further defined in the detailed description.

Typically said at least one thermo-responsive polymer may be selected from the group comprising: polyoxyethylene-polyoxypropylene block copolymers (PEO-PPO-PEO), selected from the group such as poloxamer 124, poloxamer 188, poloxamer 237, poloxamer 338, poloxamer 407 and the like, poly(ethylene glycol)/poly(lactide-coglicolide) block copolymers (PEG-PLGA), poly(ethylene glycol)-poly(lactic acid)- poly(ethylene glycol) (PEG-PLA-PEG), poly(N-isopropylacrylamide) and thermo-responsive cellulose derivatives such as methylcellulose, hydroxypropyl methyl cellulose, ethyl (hydroxyethyl) cellulose, and the like and mixtures of the above.

Typically said at least one ion sensitive polymer may be selected in the group of polysaccharides, comprising but not limited to, carrageenan, gellan gum, pectin, alginic acid and salts thereof and mixtures of the above.

Typically said at least one lipophilic compound can be selected in the group comprising, but not limited to: unsaturated or hydrogenated alcohols of fatty acids selected from the group comprising, but not limited to: stearyl alcohol, cetyl alcohol or cetearyl alcohol and the like, salts, esters or amides thereof, fatty acid mono-, di-or triglycerides, the polyethoxylated derivatives thereof, waxes, ceramides, cholesterol derivatives, stearic acid and salts thereof, palmitic acid and salts thereof, cetyl acid and salts thereof, arachidonic acid and salts thereof, behenic acid and salts thereof, and the like and mixtures thereof.

Suitable active substances for delivery belong to the therapeutic classes of small chemical compounds or biologic material, such as proteins, peptides, monoclonal antibodies, anti- TNFs, immune modulators, cytokines, chemo-cytokines, anti-neoplastics, analgesics, anti-inflammatories, antibiotics, antiadrenergic drugs, local anesthetics, non steroid anti-inflammatory drugs, steroid anti-inflammatory drugs, anti-diarrheals drugs, intestinal anti-inflammatories, peripheral opioid receptor antagonists, laxatives, spasmolytics, 3-hydroxy-3-methylglutaryl-coenzyme A (HMG-CoA) reductase inhibitors, bile acid sequestrants and are suitable for oral target administration in view of systemic activity or for acting topically at some areas of the human body, preferably of the gastrointestinal tract.

The active substances which can conveniently be formulated according to the invention comprise:
- proteins, peptides, anti-TNFs, such as anti-TNF alpha agents and monoclonal antibodies, such as colostrum, certolizumab pegol, infliximab, adalimumab, ustekinumab;
- cytokines such as alfa-1, alfa-2, beta-1 and gamma-1 interferons, interleukin-2 (IL-2), interleukin-11 (IL-11), platelet-derived growth factor (PDGF), transforming growth factor β (TGFβ)
- anti-neoplastics such as alkylating agents, antimetabolites, interferons, antineoplastic monoclonal antibodies, BCR-ABL tyrosine kinase inhibitors, EGRF inhibitors, HER2 inhibitors, mitotic inhibitors, 5-FU, paclitaxel, etoposide, flutamide, enzalutamide or the like.
- analgesics, such as acetaminophen, phenacetin, sodium salicylate;
- antibiotics, such as aminoglycosides, carbapenems, cephalosporins, penicillins, macrolides, ansamycins, tetracyclins, sulphonamides, tetracyclins;
- antiadrenergic drugs, such as doxazosin, prazosin, terazosin;
- local anesthetics, such as bupivacaine, lidocaine, mepivacaine, articaine, salts thereof, and the like;
- non steroid anti-inflammatory drugs, such as ketoprofen, ibuprofen, diclofenac, diflunisal, piroxicam, flurbiprofen, naproxen, ketorolac, nimesulide, thiaprophenic acid, mesalazine (5-aminosalicylic acid);
- steroid anti-inflammatory drugs, such as prednisolone, budesonide, hydrocortisone, betamethasone, triamcinolone, salts or esters thereof, and the like;
- anti-diarrheal drugs, such as bismuth subgallate, Loperamide and salts thereof, lactobacillus acidophilus
- intestinal anti-inflammatories, such as loperamide, 5-aminosalicylic, olsalazine, sulfasalazine, budesonide;
- peripheral opioid receptor antagonists, such as methylnaltrexone, naloxegol and the like;
- laxatives, such as magnesium salts (citrate, sulfate, phosphate bicarbonate and the like), senna, bisacodyl, lactulose, polyethylene glycols (PEGs), phosphate salts, docusate and the like;
- spasmolytics such as octylonium bromide, hyoscyamine, methscopolamine 3-hydroxy-3-methylglutaryl-coenzyme A (HMG-CoA) reductase inhibitors, such as lovastatin and simvastatin; and bile acid sequestrants, such as cholestyramine and colesevelam;

In one embodiment said at least one active substance is a peptide.

In one embodiment said at least one active substance is a protein.

In one embodiment said at least one active substance is a monoclonal antibody.

In one embodiment said at least one active substance is an immune modulator.

The invention herein disclosed provides controlled release solid pharmaceutical novel compositions containing an active ingredient, which may be either a small molecule or a biomolecule. The compositions of the invention are able to overcome the different physiological changes and variability in the GI tract that characterize a pathology, a disorder or an alteration as, for example, chronic and active inflammation in Inflammatory Bowel Disease (IBD) patients including pH, GI transit time and colonic microbiome.

The novel composition anchors the active ingredient for controlled delivery at the target site for the time suitable to obtain the maximization of the pharmacological effect.

The pharmaceutical compositions of the invention are able to provide a controlled release of the at least one drug therein contained, specifically a delayed, extended or delayed and extended release.

### DETAILED DISCLOSURE OF THE INVENTION

It has surprisingly been discovered that the combination of at least one thermo-responsive polymer, at least one ion-sensitive polymer and at least one lipophilic compound in the same composition leads to the obtainment of an improved controlled release solid formulation, typically upon oral administration. The formulation of the invention is able to target the release of the active substance at a pre-specified site of action and to ensure the release of an active ingredient at desired extent for a prolonged period of time, preferably in the gastrointestinal tract.

In one aspect, the invention herein disclosed provides a solid pharmaceutical composition, preferably an oral pharmaceutical composition for oral administration of active substances, comprising at least one active substance, at least one thermo-responsive polymer, at least one ion-sensitive polymer and at least one lipophilic excipient, and optionally other physiologically acceptable excipients.

The two polymers possess different physical-chemical characteristics and, together with said at least one lipophilic compound, act synergistically in ensuring a controlled release of said at least one active substance.

Both said thermo-responsive polymer and said ion-sensitive polymer are able to provide a system structuring in situ upon administration, i.e. to transform into gels the composition or the part of composition in contact with the body organs, upon contact with a biological medium. Said thermo-responsive polymer is able to become structured (or jellify) when the temperature is brought to body temperature, i.e. about 37°C; said ion-sensitive polymer is able to become structured (or jellify) when it comes into contact with specific inorganic ions. These peculiar characteristics have been combined in the compositions of the inventions to obtain oral pharmaceutical compositions of peculiar characteristics of target or drug release The advantage of such application consists generally in the fact that these said thermo-responsive polymer structures when the temperature is brought to body temperature, i.e. about 37°C, allowing the formulation to graft to the mucosal membrane or other cellular structures to warrant the maximization of the efficacy of the active ingredient delivered. Meanwhile, said ion-sensitive polymer is able to provide a more structured system once it comes in contact with specific ions, thereby strengthening the gel system formed by the thermo-responsive polymer.

The combination said thermo-responsive polymer and said ion-sensitive polymer produces an unexpected synergy, increasing the composition ability to interact with the human body. Even though both polymers are known to be able to jellify independently upon contact with a biological medium or upon increase of temperature up to body temperature (i.e. about 37°C), it has been surprisingly discovered that, when the 2 polymers are combined together, they act synergistically, with the thermo-responsive polymer strengthening the interaction of the ion-sensitive polymer with its bio-relevant medium, and vice-versa: as results, the composition increase the structuring power in a way more than proportional to the individual contribution and this effect could be more advantageously exploited for drug targeted and controlled release.

Upon contact with the gastro-intestinal fluids, in facts, the synergistic action of the combined stimuli-responsive polymers, which entrap the active substance inside the composition, lead to a fast jellification of the outer layer of the composition. The jellification exerts a double action on the surface of the pharmaceutical composition: it determines the creation of a highly hydrated and viscous layer at the outer surface of the composition, which slows down the penetration of further water into the composition, and it determines the creation and release of micelles at the outer surface of the layer (i.e. the layer immediately exposed to the biological medium). The formed micelles in turn contain at least part of the total amount of the active substance, which is therefore transported and spread along the gastro intestinal tract walls.

The presence of said at least one lipophilic compound in the product formulation exerts an action in further controlling the penetration of water inside the composition. In fact, due to its hydrophobic characteristics, the lipophilic compound slows down the penetration of water into the inner layer of the composition, thereby increasing the prolonged-release action of the composition of the invention.

The different combinations of the aforementioned compounds, namely said at least one thermosensitive polymer, said at least one ion-sensitive polymer and said at least one lipophilic compound, in the formulations of the invention, allows to modulate the release the active substance based on the target site of action, precisely the characteristics and surface of contact with the body organ, and on the characteristics of the active substance.

The gastrointestinal tract has conventionally been subdivided in: a) upper GI tract, comprising the buccal cavity, pharynx, esophagus, stomach, and duodenum, and b) lower GI tract, comprising most of the small intestine (jejunum and ileum) and the large intestine, rectum and anus. Depending on the desired site of action, a different release profile might be advisable.

The formulations of the invention, thanks to the synergistic action of said at least one thermosensitive polymer and at least one ion-sensitive polymer, combined with the action of said at least one lipophilic compound, can be optimized to design a release profile of the active substance customized to the desired site of action.

According to the present invention there is provided a controlled release solid composition of an active substance, the composition comprising:
at least one active substance to be delivered;
at least one thermo-responsive polymer;
at least one ion-sensitive polymer; and
at least one lipophilic compound.

In one aspect the invention herein disclosed provides a controlled release solid pharmaceutical composition comprising:
(a) at least one active substance;
(b) a hydrophilic matrix comprising a combination of at least one thermo-responsive polymer and at least one ion-sensitive polymer
(c) at least one lipophilic compound;
(d) optionally other physiologically acceptable excipients.

The composition of the invention is preferably administered orally, in a fed or fast state, in single or multiple administrations per day, to diagnose, prevent, alleviate, treat, reduce and/or induce or maintain remission of pathologies or disorders affecting the human body.

According to one aspect, the invention provides a controlled release solid pharmaceutical composition for use in the diagnosis, prevention, alleviation, treatment, reduction and/or induction or maintenance of remission of pathologies or disorders of the gastrointestinal tract, such as inflammatory bowel disease (IBD), Irritable Bowel Syndrome (IBS), Diverticular diseases, Crohn's disease, ulcerative colitis, gastrointestinal tumors, celiac disease, hemorrhoids, constipation, opioid induced constipation (OIC), idiopathic constipation (IC), diarrhea, infectious diarrhea, chronic diarrhea, collagenous colitis, microscopic colitis, small intestine bacterial overgrowth, cholera, pouchitis, meteorism, intestinal infections, familial adenomatous polyposis and the like

According to another aspect, the invention provides a controlled release solid pharmaceutical composition for use in the cleansing of the colon and/or rectum prior to endoscopic examination, such as colonoscopy, or prior to surgery.

According to another aspect, the invention provides a controlled release solid pharmaceutical composition for use in lowering the plasma levels of cholesterol.

The same results could be obtained when the invention herein disclosed provides a controlled release solid pharmaceutical composition for use in the diagnosis, prevention, alleviation, treatment, reduction and/or induction or maintenance of remission of pathologies or disorders affecting the human body, wherein said composition comprises:
(a) at least one active substance;
(b) at least one thermo-responsive;
(c) and at least one ion-sensitive polymer
(d) at least one lipophilic compound;
(e) optionally other physiologically acceptable excipients.

In another aspect, the invention herein disclosed provides a controlled release solid pharmaceutical composition and the same composition for use in the diagnosis, prevention, alleviation, treatment, reduction and/or induction or maintenance of remission of pathologies or disorders affecting the human body, wherein said composition comprises:
(a) at least one active substance;
(b) a hydrophilic matrix comprising a combination of at least one thermo-responsive and at least one ion-sensitive polymer
(c) at least one lipophilic compound;
(d) optionally other physiologically acceptable excipients.

In one embodiment, the composition of the invention is formulated in form of tablets.

In another embodiment, the composition of the invention is formulated in form of mini-tablets.

In another embodiment, the composition of the invention is formulated in form of capsules.

In another embodiment, the composition of the invention is formulated in form of granules or pellets.

In a preferred embodiment, the pharmaceutical composition of the invention is formulated in form of tablets.

According to the invention, a thermo-responsive polymer can be selected among those able to exhibit temperature dependence of the sol-gel transitions upon contact with water or biorelevant medium. Such thermo-responsive macromolecular compound is activated by the body temperature (i.e. about 37°C). This stimulus causes a reaction by changing the molecular interactions between the polymer and solvent or between polymer's chains. Variations of these behaviors may include changes in solubility, hydrophilic/lipophilic balance and conformation.

According to the invention herein disclosed, said at least one thermo-responsive polymer may be selected in the group comprising, but not limited to: polyoxyethylene-polyoxypropylene block copolymers (PEO-PPO-PEO), poly(ethylene glycol)/poly(lactide-coglicolide) block copolymers (PEG-PLGA), poly(ethylene glycol)-poly(lactic acid)- poly(ethylene glycol) (PEG-PLA-PEG), poly(N-isopropylacrylamide) and thermo-responsive cellulose derivatives.

Any mixture of the above thermo-responsive polymers can be used to form the appropriate solid composition. In some embodiments, said at least one thermo-responsive polymer is a polyoxyethylene-polyoxypropylene block copolymers (PEO-PPO-PEO), selected in the group comprising, but not limited to poloxamer 124, poloxamer 188, poloxamer 237, poloxamer 338, poloxamer 407 and the like.

In an embodiment, said at least one thermo-responsive polymer is poloxamer 188.

In another embodiment, said at least one thermo-responsive polymer is poloxamer 407.

Further in another embodiment, said at least one thermo-responsive polymer is a mixture of poloxamer 188 and poloxamer 407. In a preferred embodiment, said at least one thermo-responsive polymer is poloxamer 407.

In some embodiments, said at least one thermo-responsive polymer is a cellulose derivative, selected from the group comprising, but not limited to: methylcellulose, hydroxypropyl methyl cellulose, ethyl (hydroxyethyl) cellulose, and the like.

In an embodiment, said at least one thermo-responsive polymer is Hydroxypropylmethylcellulose. In another embodiment embodiment, said at least one thermo-responsive polymer is methylcellulose.

Further in another preferred embodiment, said at least one thermo-responsive polymer is a mixture of poloxamer 407 and hydroxypropylmethylcellulose.

Any mixture of the above thermo-responsive polymers can be used to form the appropriate solid composition.

According to the invention herein disclosed, the amount of said at least one thermo-responsive polymer ranges between about 0.1% to about 50% by weight with respect to the weight of the composition, preferably between about 1 % to about 50% by weight with respect to the weight of the composition preferably between about 5 % to about 40% by weight with respect to the weight of the composition, more preferably between about 10% to about 35% by weight with respect to the weight of the composition, much more preferably between about 20% and about 30% by weight with respect to the weight of the composition.

In some embodiments, said at least one thermo-responsive polymer is contained in an amount of about 0.1% or about 1% or about 5 % or about 10% or about 20% or about 30% by weight with respect to the weight of the solid composition.

According to the invention, an ion-sensitive polymer can be selected among those able to bind mono and/or divalent inorganic ions, such as Na⁺, K⁺, Mg²⁺, Ca²⁺, Zn²⁺or organic cations, with high affinity. The responsiveness to ionic strength is a typical property of polymers containing ionizable groups.

Changes in ionic strength are typical in the GI tract and may cause changes in the size of the polymeric micelles and polymer solubility. Different concentration of salts, which determine the ionic strength, may cause phase transition in ionic strength responsive polymers.

The combination of said thermo-responsive polymer and ion-sensitive polymer, produces an unexpected synergy, increasing their ability to interact with the human body, strengthening the interaction with the environmental medium each of the other, resulting in an expansion of the mixture capability of jellifying. The combination of said polymers in the compositions of the invention therefore modulates the release of the at least one active at the desired site, enhancing the composition bio-adhesion and hence its permanence time at the site.

Additionally, it has been discovered that the combination of the polymers of the invention can maintain the pH value inside the polymer network constant, contributing to the increase in stability of the incorporated drug by limiting its pH-dependent degradation. Hydrated ionic polymers can maintain the pH value of around 7 in the intestine protecting the embedded macromolecular drugs inside the swollen matrix from degradation for several hours.

According to the invention herein disclosed, said at least one ion sensitive polymer may be selected in the group of polysaccharides, comprising, but not limited to carrageenan, gellan gum, pectin, alginic acid and salts thereof and mixtures of the above.

In an embodiment, said at least one ion sensitive polymer is alginic acid or a salt thereof. In another embodiment, said at least one ion sensitive polymer is sodium alginate or a salt thereof.

In a preferred embodiment, said at least one ion-sensitive polymer is gellan gum. Gellan gum is a polysaccharide resistant to gastric enzymes, but metabolized by anaerobic bacteria in the intestine and in particular in the colon, thus enhancing controlled release properties of the invention.

According to the invention herein disclosed, said at least one ion-sensitive polymer is contained in an amount which ranges from about 0.1% to about 40% by weight with respect to the weight of the solid composition, preferably from about 1 % to about 30 % by weight with respect to the weight of the solid composition, more preferably from about 5% to about 25% by weight with respect to the weight of the solid composition.

According to a preferred aspect, said at least one ion-sensitive polymer is contained in an amount of about 0.1% or about 5 % or about 20% or about 30% by weight with respect to the weight of the solid composition.

According to the invention, said at least one lipophilic compound can be selected in the group comprising, but not limited to: unsaturated or hydrogenated alcohols or fatty acids, salts, esters or amides thereof, fatty acids mono-, di-or triglycerids, the polyethoxylated derivatives thereof, waxes, ceramides, cholesterol derivatives or mixtures thereof.

In some embodiments, said at least one lipophilic compound is a fatty alcohol, selected from the group comprising, but not limited to: stearyl alcohol, cetyl alcohol or cetearyl alcohol and the like.

In other embodiments, said at least one lipophilic compound is a fatty acid or a salt thereof, selected from the group comprising, but not limited to: stearic acid and salts thereof, palmitic acid and salts thereof, cetyl acid and salts thereof, arachidonic acid and salts thereof, behenic acid and salts thereof, and the like.

Any physiologically acceptable salt, of organic or inorganic nature, of the above fatty acid may be used to form the composition of the invention.

By way of example, physiologically acceptable salts comprise sodium salts, potassium salts, calcium salts, magnesium salts, zinc salts and the like. In other embodiments, said at least one lipophilic compound is a fatty acid ester, selected from the group comprising, but not limited to: stearyl esters, palmityl esters, cetyl esters, arachidoyl esters, glyceryl behenate and the like.

Any physiologically acceptable alcohol can be used to form the above fatty acid esters; by way of example, physiologically acceptable alcohols comprise ethyl alcohol, isopropyl alcohol, propyl alcohol, fatty acid alcohols and the like.

In other embodiments, said at least one lipophilic compound is an ester of a fatty alcohol with a low molecular weight organic acid, selected from the group comprising, but not limited to: fatty alcohol-acetate esters, fatty alcohol-propionate esters, fatty alcohol-butyrate esters, fatty alcohol-citrate mono-esters and salts thereof, fatty alcohol-maleate mono-esters and salts thereof, fatty alcohol-fumarate mono-esters and salts thereof, and the like.

Any mixture of the above lipophilic compounds may be used to form the appropriate composition according to the invention.

In an embodiment, said at least one lipophilic compound is stearic acid.

In another embodiment, said at least one lipophilic compound is sodium stearate.

In another embodiment, said at least one lipophilic compound is magnesium stearate.

In another embodiment, said at least one lipophilic compound is sodium stearyl fumarate.

In a preferred embodiment, said at least one lipophilic compound is stearic acid.

In another preferred embodiment, said at least one lipophilic compound is magnesium stearate

In another preferred embodiment, said at least one lipophilic compound is glyceryl behenate.

In another embodiment the release of the active substance can be modulated by the addition of a resin in which the hydrophilic matrix associated with at least one lipophilic compound is dispersed. Upon contact with the gastro-intestinal fluids the resin can be cross-linked to form gels that are highly water-retentive. Then, due to the presence of the aqueous solvent, the matrix swells due to the macromolecular relaxation or distension of the polymeric chains of the hydrogels, giving rise to a high viscosity hydrated front which prevents the further penetration of the solvent itself linearly slowing down the dissolution process independently on the pH.

Therefore, in another aspect the invention herein disclosed provides a solid controlled release pharmaceutical composition comprising:
(a) at least one active substance;
(b) at least one thermo-responsive;
(c) at least one ion-sensitive polymer;
(d) at least one lipophilic compound;
(e) at least one resin;
(f) optionally other physiologically acceptable excipients.

In another aspect the invention herein disclosed provides a controlled release solid pharmaceutical composition comprising:
(a) at least one active substance;
(b) a hydrophilic matrix comprising a combination of at least one thermo-responsive and at least one ion-sensitive polymer;
(c) at least one lipophilic compound;
(d) at least one resin;
(e) optionally other physiologically acceptable excipients.

In another aspect the invention herein disclosed provides a controlled release solid pharmaceutical composition for use in the diagnosis, prevention, alleviation, treatment, reduction and/or induction or maintenance of remission of pathologies or disorders affecting the human body, wherein said composition comprising:
(a) at least one active substance;
(b) at least one thermo-responsive;
(c) at least one ion-sensitive polymer;
(d) at least one lipophilic compound;
(e) at least one resin;
(f) optionally other physiologically acceptable excipients.

In another aspect, the invention herein disclosed provides a controlled release solid pharmaceutical composition for use in the diagnosis, prevention, alleviation, treatment, reduction and/or induction or maintenance of remission of pathologies or disorders affecting the human body, wherein said composition comprising:
(a) at least one active substance;
(b) a hydrophilic matrix comprising a combination of at least one thermo-responsive and at least one ion-sensitive polymer;
(c) at least one lipophilic compound;
(d) at least one resin;
(e) optionally other physiologically acceptable excipients.

According to the invention, said at least one resin is selected but not limited to nonionic poly-(ethylene oxide) polymers, commercially known as Polyox™; Polacrilin resins, Colestyramine; anion-exchange resins and cation-exchange resins.

According to a preferred embodiment, said at least one resin is selected but not limited to nonionic poly-(ethylene oxide) polymers, commercially known as Polyox™.

According to the invention, said at least one resin is contained in an amount ranging from about 0.1% to about 30% by weight with respect to the weight of the solid composition, preferably from about 1 % to about 25% by weight with respect to the weight of the solid composition, more preferably from about 2% to about 20% by weight with respect to the weight of the solid composition.

The solid composition of the invention herein disclosed may further comprise at least one additional bio-adhesive compound.

Therefore, in another aspect the invention herein disclosed provides a controlled release pharmaceutical composition comprising:
(a) at least one active substance;
(b) at least one thermo-responsive;
(c) at least one ion-sensitive polymer;
(d) at least one lipophilic compound;
(e) at least one bio-adhesive compound;
(f) optionally at least one resin;
(g) optionally other physiologically acceptable excipients.

In another aspect, the invention herein disclosed provides a controlled release solid pharmaceutical composition comprising:
(a) at least one active substance;
(b) a hydrophilic matrix comprising a combination of at least one thermo-responsive and at least one ion-sensitive polymer;
(c) at least one lipophilic compound;
(d) at least one bio-adhesive compound
(e) optionally, at least one resin;
(f) optionally other physiologically acceptable excipients.

In another aspect the invention herein disclosed provides a controlled release solid pharmaceutical composition for use in the diagnosis, prevention, alleviation, treatment, reduction and/or induction or maintenance of remission of pathologies or disorders affecting the human body, wherein said composition comprising:
(a) at least one active substance;
(b) at least one thermo-responsive;
(c) at least one ion-sensitive polymer;
(d) at least one lipophilic compound;
(e) at least one bio-adhesive compound;
(f) optionally at least one resin;
(g) optionally other physiologically acceptable excipients.

In a further aspect the invention herein disclosed provides a controlled release solid pharmaceutical composition for use in the diagnosis, prevention, alleviation, treatment, reduction and/or induction or maintenance of remission of pathologies or disorders affecting the human body, wherein said composition comprising:
(a) at least one active substance;
(b) a hydrophilic matrix comprising a combination of at least one thermo-responsive and at least one ion-sensitive polymer;
(c) at least one lipophilic compound;
(d) at least one bio-adhesive compound
(e) optionally at least one resin;
(f) optionally other physiologically acceptable excipients.

According to the invention herein disclosed, said bio-adhesive compound may be selected from the group comprising, but not limited to: chitosan, cellulose derivatives, such methyl cellulose, hydroxy propyl methylcellulose, hydroxy propyl cellulose, carboxymethyl cellulose sodium and the like, polyvinyl alcohol, polyvinyl pyrrolidone, polycarbophil, polyacrylic acid, poly ethylene oxides, tragacanth, xanthan gum, guar gum, gelatin, dextran, hyaluronic acid and salts thereof, glyceryl monooleate, cyclodextrins and the like.

Any mixture of the above bio-adhesive compounds may be used to obtain suitable pharmaceutical solid compositions according to the invention.

In an embodiment, said at least one bio-adhesive compound is xanthan gum. In another embodiment, said at least one bio-adhesive compound is polycarbophil.

In a preferred embodiment, said at least one bio-adhesive compound is carboxymethyl cellulose sodium.

In another preferred embodiment, said at least one bio-adhesive compound is hyaluronic acid and/or a salt thereof.

According to these embodiments, said at least one bio-adhesive polymer provides an additional synergy with the said at least one thermo-responsive polymer, said at least one ion-sensitive polymer and said at least one lipophilic compound, leading to an improved level of adhesion of the solid vehicle and an improved residence time at the desired site.

According to these embodiments, the efficacy of the active substance contained in the solid delivery vehicle can be maximized.

According to the invention herein disclosed, said at least one bioadhesive compound is contained in an amount which ranges from about 0.05% to about 10% by weight with respect to the weight of the solid composition, preferably from about 0.5% to about 5 % by weight with respect to the weight of the solid composition, more preferably from about 1% to about 2% by weight with respect to the weight of the solid composition.

According to a preferred embodiment, said at least one ion-sensitive polymer is contained in an amount of about 0.5% or about 1 % or about 2% or about 10% by weight with respect to the weight of the solid composition.

In a preferred embodiment said at least one active substance is a peptide.

In a preferred embodiment said at least one active substance is a protein.

In a preferred embodiment said at least one active substance is a monoclonal antibody.

In a preferred embodiment said at least one active substance is a cytokine.

In a preferred embodiment said at least one active substance is a growth factor.

In a preferred embodiment said at least one active substance is an anti TNF agent, preferably an anti TNF-alpha antibody.

In a preferred embodiment said at least one active substance is an immune modulator.

In a preferred embodiment the active substance is a mixture of the above biologic compounds.

The combination of the invention comprising at least one thermo-responsive and at least one ion-sensitive polymer dispersed in at least one lipophilic compounds can be particularly useful when the active substance in the composition is a peptide, protein, immune modulator or monoclonal antibody where smart copolymers thermodynamically stabilize self-assembling lipophilic colloids to protein and peptide delivery.

According to the invention, said at least one physiologically acceptable excipients can include one or more enzymatic inhibitor like but not limited to: EDTA and salts thereof, DTPA, polyacrylic acid, thiomers, sodium alginate, chitosan-EDTA and salts thereof and the like.

In a preferred embodiment said at least one enzymatic inhibitor is EDTA and salts thereof. EDTA complexes divalent ions inhibiting the conversion of trypsinogen in trypsin and reducing trypsin proteinase activity. This complexation stabilizes peptides, protein, immune modulator, monoclonal antibody that are known to be very sensitive to hydrolytic action of the trypsin.

According to the invention, said at least one physiologically acceptable excipients can include one or more protein stabilizer selected form the group comprising, but not limited to: polyols (mannitol, sorbitol), sugars (Sucrose, glucose and trehalose), amino acids, amines, and salting out salts or combination thereof.

In a preferred embodiment said protein stabilizer is an amino acid, selected from the group comprising, but not limited to: histidine, arginine, and glycine, methionine, proline, lysine, glutamic acid, arginine and the like, and mixtures thereof.

According to the invention, said at least one physiologically acceptable excipients can include one or more competitive inhibitor of proteolytic enzymes, selected form the group comprising, but not limited to: albumin, collagen, colostrum, (hydrolyzed) soy protein. According to such an embodiment, one or more competitive inhibitor leads to the obtainment of an improved stabilization of the active ingredient that are sensible to the proteolytic enzymatic hydrolysis. Proteins, peptides and monoclonal antibodies are known to be hydrolyzed by this proteolytic enzymes and the addition of one or more competitive or non-competitive inhibitor to the compositions of the invention allows the decrease of the hydrolytic action of these enzymes on the active ingredient to be delivered.

According to the invention, said at least one physiologically acceptable excipients can include one or more buffering agents selected form the group comprising, but not limited to: TRIS, histidine, citrate, phosphate.

Additional excipients useful in the preparation of the compositions of the invention are well known in the art, and comprise, by way for example: glidants, lubricants, fillers, disintegrants, antioxidants, and the like.

The solid compositions of the invention can be obtained by manufacturing processes well known in the art. If shaped as a capsule, the physical state could be a powder or a freeze-dried powder mixture in a gelatin capsule shell, if shaped as tablet, it could be manufactured through the standard processes of mixing and direct compression, or wet or dry granulation followed by compression, and optional film coating.

Because the proteolytic enzymes are distributed in the human body according to a negative gradient from the stomach to the rectum, in order to protect the transit of the composition through the first GI districts (namely stomach, duodenum and jejunum, the most part of the solid compositions obtainable according to the invention, especially tablets, can optionally be subjected to known coating processes with a gastro-resistant or enteric -resistant film, consisting of, for example, methacrylic acids polymers (Eudragit (R)) or cellulose derivatives, such as cellulose acetophthalate, ethyl cellulose.

The film coating composition may vary according the target site of action and disorder to be treated in order to better target the start of drug release.

In a preferred embodiment a methacrylic acid polymers Eudragit L, Eudragit S, Eudragit FS and Eudragit E or combination thereof could be used in different ratios to form a coating with an optimized intestinal release.

In another preferred embodiment a mixture of methacrylic acid polymers, Eudragit L, Eudragit S, Eudragit FS and Eudragit E or combination therof, and gellan gum could be used in order to guarantee the gastro resistance and colon target delivery overcoming the pH fluctuations along the gastro intestinal tract that could occur especially in IBD patients.

The amphiphilic compounds which can be used according to the invention comprise polar lipids of type I or II (lecithin, phosphatidylcholine, phosphatidylethanolamine), ceramides, glycol alkyl ethers.

The hydrophilic matrix consists of excipients known as hydrogels, i. e. substances which when passing from the dry state to the hydrated one, undergo the so-called"molecular relaxation", namely a remarkable increase in mass and weight following the coordination of a large number of water molecules by the polar groups present in the polymeric chains of the excipients themselves.

Examples of hydrogels which can be used according to the invention are compounds selected from acrylic or methacrylic acid polymers or copolymers, alkylvinyl polymers, hydroxyalkyl celluloses, carboxyalkyl celluloses, polysaccharides, dextrins, pectins, starches and derivatives, natural or synthetic gums, alginic acid.

The compositions of the invention can contain more than one active ingredient and are preferably in the form of tablets, capsules or mini-tablets.
In a typical application of this invention, a pharmaceutical controlled release composition is formulated as a tablet, contains a protein and is coated with a gastro-protective film able to bring the administered tablet to an environment when the pH has the characteristics to solubilize the film coating layer. At that point, the tablet starts to swell in a controlled way, releasing polymer micelles that contain at least part of the total amount of the active ingredient and are spread onto the luminal layer of the intestinal mucosa, where, through a bioadhesive force, the micelles are grafted. In such situation the contact between the composition and the mucosa is enhanced for a suitable time to allow the drug contained into the micelles to be absorbed by the intestinal mucosal lining cells and internalized toward systemic circulation or used locally for a topical pharmacological activity. The time of contact between the mucosal cells and the composition bioadhered can be protracted till the cell physiological turnover is reached or till the dilution of the composition layered to the lining mucosa will make the gelled composition able to develop a bioadhesive force to the mucosal cell.

### EXAMPLES

The invention is exemplified in the following non-limiting examples of formulations.

### Example 1:

| Component(s) | Unit | Amount per tablet |
|---|---|---|
| Core composition | | |
| Colostrum 60-201 ⁽¹⁾ | mg | 25,08 |
| Hydroxypropyl methyl cellulose (Methocel K15M) | mg | 33,00 |
| Gellan gum | mg | 22,00 |
| Poloxamer 407 | mg | 49,94 |
| Colloidal Silicon Dioxide | mg | 2,20 |
| Lecithin | mg | 2,20 |
| Glyceryl behenate | mg | 15,40 |
| Tris | mg | 5,50 |
| Dibasic calcium phosphate | mg | 26,18 |
| Cellulose microcrystalline | mg | 26,18 |
| Magnesium stearate | mg | 2,20 |
| Xanthan Gum | mg | 4,40 |

| Coating composition | | |
|---|---|---|
| Eudragit L100 | mg | 4,80 |
| Eudragit S100 | mg | 4,80 |
| Triethyl citrate | mg | 0,96 |
| Talc | mg | 4,44 |
| Ethanol ⁽²⁾ | | ------ |

| | | |
|---|---|---|
| (1) equal to 5.0 mg IgG (2) Ethanol is essentially removed during manufacturing process. | | |

Colostrum and Poloxamer were mixed in a suitable container till homogeneity of dispersion was obtained. Then hydroxypropyl methyl cellulose, gellan gum, lecithin, glyceryl behenate, TRIS, cellulose microcrystalline, dibasic calcium phosphate, xanthan gum and silica were added and the mixture was mixed again; finally magnesium stearate was added and mixed till homogeneity, then the powder was compressed into a rotating tableting machine to obtain the tablet cores. These latter were then coated in a pan coat unit with an alcoholic mixture of acrylic acid and methacrylic copolymers, triethyl citrate, and talc.

### Example 2:

| Component(s) | Unit | Amount per tablet |
|---|---|---|
| Core composition | | |
| Certolizumab pegol | mg | 5.0 |
| (Hydroxypropyl methyl cellulose) Methocel K15M | mg | 33.0 |
| Gellan gum | mg | 22.0 |
| Poloxamer 407 | mg | 50.0 |
| Colloidal silicon dioxide | mg | 2.2 |
| Lecithin | mg | 2.2 |
| Glyceryl behenate | mg | 15.5 |
| Tris | mg | 5.5 |
| Dibasic calcium phosphate | mg | 39.0 |
| Cellulose, microcrystalline | mg | 39.0 |
| Magnesium stearate | mg | 2.2 |
| Xanthan Gum | mg | 4.4 |

| Coating composition | | |
|---|---|---|
| Eudragit L100 | mg | 8.0 |
| Eudragit S100 | mg | 8.0 |
| Triethyl citrate | mg | 1.6 |
| Talc | mg | 7.9 |
| Titanium dioxide | mg | 4.5 |
| Ethanol ⁽¹⁾ | | ------ |

| | | |
|---|---|---|
| (1) Ethanol is essentially removed during manufacturing process | | |

A quantity of 12 mL coming from a drug on the market (Cimzia®), containing 200 mg of Certolizumab Pegol in 1 mL was absorbed on a mixture of dibasic calcium phosphate, cellulose microcrystalline and colloidal silicon dioxide. The moist mixture was subjected to drying in a low temperature ventilated oven for 2 hours. The dried granulate was then sieved and then mixed with Poloxamer in a suitable container till homogeneity of dispersion was obtained. Then hydroxypropyl methyl cellulose, gellan gum, lecithin, glyceryl behenate, TRIS, xanthan gum were added and it was mixed again; finally, magnesium stearate was added and mixed till homogeneity, then the powder was compressed into a rotating tableting machine to obtain the tablet cores. These latter were then coated in a pan coat unit with an alcoholic mixture of acrylic acid and methacrylic copolymers, triethyl citrate, talc and titanium dioxide.

### Example 3:

| Component(s) | Unit | Amount per tablet |
|---|---|---|
| Core composition | | |
| Remicade® (equal to 5.0 mg Infliximab) | mg | 30.0 |
| Albumin | mg | 10.0 |
| Gellan gum | mg | 225.0 |
| (Hydrxypropylmethyl cellulose) Methocel K100 MP CR | mg | 135.0 |
| Glyceril monostearate | mg | 45.0 |
| EDTA | | 10.0 |
| Mannitol | mg | 125.0 |
| Cellulose, microcrystalline | mg | 293.0 |
| Magnesium stearate | mg | 9.0 |
| Hydroxypropyl cellulose | mg | 18.0 |

| Coating composition | | |
|---|---|---|
| Eudragit L100 | mg | 10.0 |
| Eudragit S100 | mg | 10.0 |
| Triethyl citrate | mg | 2.0 |
| Talc | mg | 10.0 |
| Gellan gum | mg | 2.0 |
| Titanium dioxide | mg | 5.8 |
| Ethanol ⁽¹⁾ | | ------ |

| | | |
|---|---|---|
| (1) Ethanol is essentially removed during manufacturing process | | |

A quantity of lyophilized powder coming from a drug on the market (Remicade®), containing 100 mg of Infliximab dispersed in 0.500 g of inert support was mixed with Poloxamer in a suitable container till homogeneity of dispersion was obtained. Then hydroxypropyl methyl cellulose, gellan gum, glyceryl monostearate, EDTA, cellulose microcrystalline, mannitol, Hydroxypropyl cellulose and albumin were added mixed again; finally, magnesium stearate was added and mixed till homogeneity, then the powder was compressed into a rotating tableting machine to obtain the tablet cores. These latter were then coated in a pan coat unit with an alcoholic mixture of acrylic acid and methacrylic copolymers, triethyl citrate, gellan gum, talc and titanium dioxide.

### Example 4:

| Component(s) | Unit | Amount per tablet |
|---|---|---|
| Core composition | | |
| Colostrum 60-20l⁽¹⁾ | mg | 80.0 |
| Gellan gum | mg | 140.0 |
| Poloxamer 407 | mg | 140.0 |
| Polyox | mg | 70.0 |
| Lecithin | mg | 7.0 |
| Glyceryl behenate | mg | 21.0 |
| Dibasic calcium phosphate | mg | 118.0 |
| Cellulose, microcrystalline | mg | 103.0 |
| Magnesium stearate | mg | 7.0 |
| Xanthan Gum | mg | 14.0 |
| Coating composition | | |
| Eudragit L100 | mg | 8.0 |
| Eudragit S100 | mg | 8.0 |
| Triethyl citrate | mg | 1.6 |
| Talc | mg | 7.9 |
| Titanium dioxide | mg | 4.5 |
| Ethanol ⁽²⁾ | | ------ |

| | | |
|---|---|---|
| (1) equal to 16.0 mg IgG (2) Ethanol is essentially removed during manufacturing process. | | |

Colostrum and Poloxamer and Polyox were mixed in a suitable container till homogeneity of dispersion was obtained. Then gellan gum, lecithin, glyceryl behenate, cellulose microcrystalline, dibasic calcium phosphate and xanthan gum were added and the mixture was mixed again; finally magnesium stearate was added and mixed till homogeneity, then the powder was compressed into a rotating tableting machine to obtain the tablet cores. These latter were then coated in a pan coat unit with an alcoholic mixture of acrylic acid and methacrylic copolymers, triethyl citrate, talc and titanium dioxide.

The invention is not limited to the embodiment(s) described herein but can be amended or modified without departing from the scope of the present invention.

## Claims

1. A solid composition for targeted and controlled release of an active substance, the composition comprising:
at least one active substance to be delivered;
at least one thermo-responsive polymer;
at least one ion-sensitive polymer; and
at least one lipophilic compound.

2. A solid composition as claimed in claim 1 comprising:
at least one active substance to be delivered; and
a hydrophilic matrix comprising a combination of the at least one thermo-responsive polymer and the at least one ion-sensitive polymer dispersed in the at least one lipophilic compound.

3. A solid composition as claimed in claim 1 or claim 2 further comprising a resin.

4. A solid composition as claimed in any of the preceding claims in the form of a tablet, mini-tablet, capsule, granules or other solid composition form.

5. A solid composition as claimed in any of the preceding claims wherein the composition further comprises one or more other optional substituents including any or all of bio-adhesive polymers, an amphiphilic compound, a gastric resistant coating and other excipients.

6. A solid composition as claimed in any of the preceding claims wherein the at least one thermo-responsive polymer is selected from the group consisting of polyoxyethylene-polyoxypropylene block copolymers (PEO-PPO-PEO) selected from the group consisting of poloxamer 124, poloxamer 188, poloxamer 237, poloxamer 338, poloxamer 407, poly(ethylene glycol)/poly(lactide-coglicolide) block copolymers (PEG-PLGA), poly(ethylene glycol)-poly(lactic acid)- poly(ethylene glycol) (PEG-PLA-PEG), poly(N-isopropylacrylamide) and thermo-responsive cellulose derivatives selected from the group consisting of methylcellulose, hydroxypropyl methyl cellulose, ethyl (hydroxyethyl) cellulose, and mixtures of the above.

7. A composition as claimed in any of the preceding claims wherein the at least one ion sensitive polymer is selected from the group of polysaccharides, consisting of carrageenan, gellan gum, pectin, alginic acid and salts thereof, and mixtures of the above.

8. A composition as claimed in any of the preceding claims wherein the at least one lipophilic compound is selected from the group consisting of unsaturated or hydrogenated alcohols of fatty acids selected from the group comprising, but not limited to: stearyl alcohol, cetyl alcohol or cetearyl alcohol, salts, esters or amides thereof, fatty acid mono-, di-or triglycerides, the polyethoxylated derivatives thereof, waxes, ceramides, cholesterol derivatives, stearic acid and salts thereof, palmitic acid and salts thereof, cetil acid and salts thereof, arachidic acid and salts thereof, behenic acid and salts thereof, and mixtures thereof.

9. A composition as claimed in any of the preceding claims wherein the at least one active substances for delivery belongs to the therapeutic classes of proteins, peptides, monoclonal antibodies, anti- TNFs, immune modulators, cytokines, chemo-cytokines, anti-neoplastics, analgesics, anti-inflammatories, antibiotics, antiadrenergic drugs, local anhestetics, -non steroid anti-inflammatory drugs, steroid anti-inflammatory drugs, anti-diarrheals drugs, intestinal anti-inflammatories, peripheral opioid receptor antagonists, laxatives, spasmolytics, 3-hydroxy-3-methylglutaryl-coenzyme A (HMG-CoA) reductase inhibitors, bile acid sequestrants and are suitable for oral target administration in view of systemic activity or for acting topically at some areas of the human body, preferably of the gastrointestinal tract.

10. A composition as claimed in any of the preceding claims wherein the at least one active substances for delivery is a protein, a peptide, a monoclonal antibody, an anti-TNFa antibody or an immune modulator.

11. A solid composition as claimed in any of the preceding claims wherein the at least one active substance for delivery is Infliximab or adalimumab or certolizumab pegol or their biosimilar compounds.

12. A composition as claimed in any of the preceding claims for use in the diagnosis, prevention, alleviation, treatment, reduction and/or induction or maintenance of remission of pathologies or disorders affecting the body.

13. A solid composition as claimed in any of the preceding claims for oral administration.

14. A solid composition as claimed in any of the preceding claims in tablets, mini-tablets, capsules, granules or pellets.
